# EUROPÄISCHE PATENTSCHRIFT

(11) **EP 0 391 233 B1**
(45) Veröffentlichungstag und Bekanntmachung des Hinweises auf die Patenterteilung: **25.10.1995**
(21) Anmeldenummer: 90105931.1
(22) Anmeldetag: 28.03.1990
(51) Int. Cl.: A61B 17/39, A61M 1/16

(54) **Elektrochirurgisches Hochfrequenzgerät**
HF electrosurgical apparatus
Appareil pour l'électro-chirurgie à haute fréquence

(30) Priorität: 07.04.1989 DE 3911416
(43) Veröffentlichungstag der Anmeldung: 10.10.1990
(73) Patentinhaber: DELMA ELEKTRO-UND MEDIZINISCHE APPARATEBAU GESELLSCHAFT mbH, D-78532 Tuttlingen (DE)
(72) Erfinder: Hagen, Alfred, D-7200 Tuttlingen 16 (DE)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- DE-B- 2 801 833
- GB-A- 897 961

## Beschreibung

Die Erfindung betrifft ein elektrochirurgisches Hochfrequenzgerät nach dem Oberbegriff des Anspruchs 1.

Bei derartigen elektrochirurgischen Hochfrequenzgeräten ist es allgemein bekannt, einen Antifaradisationskondensator zwischen dem HF-Generator und einer Aktivelektrode zum Schneiden des Gewebes vorzusehen. Die DE-PS 28 01 833 schlägt vor, die Anschlußklemmen des Antifaradisationskondensators an entsprechende Eingänge eines Verstärkers anzulegen, der ein Spannungssignal an einen Eingang einer Differenzschaltung liefert, deren anderer Eingang von einer Referenzspannung beaufschlagt ist. Die Differenzspannung erzeugt in Abhängigkeit von den anliegenden Spannungen ein Steuersignal für den HF-Generator. Dabei ist ein Eingang des Regelkreises jedoch galvanisch direkt mit dem HF-Generator verbunden.

Die am Antifaradisationskondensator abgegriffene Spannung schafft aber nicht bei allen Betriebszuständen ein brauchbares Signal zur einwandfreien Regelung des HF-Generators und damit zur Regelung des zum Schneiden erforderlichen Lichtbogens. Zum anderen kann es aber überdies bei einem Kurzschluß am Regelkreiseingang auch zu einer galvanischen Verbindung zwischen Patient und dem den Patienten mit Spannung beaufschlagenden Spannungsgenerator kommen, was zu Gefährdungen des Patienten und des Personals führt.

Der Antifaradisationskondensator wird nach einer anderen Ausführungsform der DE 28 01 833 durch eine elektronisch von der an der Schneidstelle entstehenden Gleichspannung gesteuerte Gleichspannungsquelle ersetzt, die dieser Gleichspannung zur Verhinderung eines unerwünschten Faradisationsstromes entgegenwirkt und als Regelgröße unmittelbar die an der Schneidstelle entstehende Gleichspannung benützt. Es liegt somit von vorneherein eine unerwünschte galvanische Verbindung zwischen dem Patienten und dem HF-Generator vor.

Aus der GB-A-897961 ist eine Vorrichtung bekannt, mit der ein elektrochirurgisches Hochfrequenzgerät beim Anlegen der aktiven Elektrode an den Patienten automatisch angeschaltet werden werden kann. Hierzu wird zwischen die aktive Elektrode und die Neutralelektrode bei zunächst abgeschaltetem HF-Generator eine Hilfsspannung angelegt. Wenn die aktive Elektrode den Patienten berührt, wird in der Vorrichtung der GB-A-897961 ein Gleichspannungsweg für diese Hilfsspannung geschlossen. Der durch die Hilfsspannung verursachte und durch den Patienten geführte Stromfluß führt an einem im Hilfsschaltkreis vorgesehenen Widerstand zu einem Spannungsabfall, und auf den Nachweis dieses Spannungsabfalls hin wird der HF-Generator angeschaltet. Bei der bekannten Vorrichtung ist aber eine auch bei Beabsichtigung nur geringer Gleichströme problematische galvanische Verbindung zwischen dem Hilfsspannungsgenerator und dem Patienten funktionsnotwendig.

Die Aufgabe der vorliegenden Erfindung besteht demnach darin, ein baulich einfaches elektrochirurgisches Hochfrequenzgerät der eingangs genannten Art zu schaffen, dessen Ausgangsleistung ohne das Erfordernis oder die Gefahr einer galvanischen Verbindung zwischen Gleichspannung führenden Teilen und dem Patienten, also auf sichere Weise, automatisch geregelt ist, um so stets einen zum Schneiden von Gewebe geeigneten Lichtbogen zu erhalten.

Diese Aufgabe wird erfindungsgemäß durch die kennzeichnenden Merkmale des Anspruchs 1 gelöst.

Durch die erfindungsgemäße Verbindung einer proportionalen Entladeerkennungsschaltung, deren Ausgang mit einem Eingang einer den HF-Generator steuernden Vergleichsspannung verbunden ist, mit den Elektroden, also mit der für chirurgische Eingriffe vorgesehenen Aktivelektrode und der Neutralelektrode wird erreicht, daß eine infolge unsymmetrische Entladevorgänge im HF-Kreis auftretende Gleichspannung zwischen den Elektroden einwandfrei erfaßt und zur Regelung des HF-Generators nutzbar gemacht wird, ohne daß eine galvanische Verbindung zwischen den Gleichspannung führenden Teilen und dem Patienten vorliegt oder durch einen Kurzschluß im Eingangskreis der Regelschaltung hervorgerufen werden kann.

Die Entladeerkennungsschaltung umfaßt in einer bevorzugten Ausführung erfindungsgemäß eine stromkompensierte Drossel. Durch diese baulich einfache Maßnahme wird erreicht, daß die Entladeerkennungsschaltung eine hervorragende Sperre für hochfrequente Schwingungen bildet, da die Impedanz einer stromkompensierten Drossel bei unsymmetrischen Schwingungen voll wirksam ist, und eine sehr glatte Gleichspannung geliefert wird, die sich proportional zur Stärke des zwischen dem Gewebe und der Aktivelektrode gebildeten Lichtbogens ändert.

Somit läßt sich die an der Ausgangsseite der Entladeerkennungsschaltung angreifbare Spannung einwandfrei zur Steuerung bzw. Regelung des HF-Generators verwenden.

Bei einer Weiterbildung der Erfindung ist vorgesehen, daß die Vergleichsschaltung ein einstellbarer Schwellwertschalter ist, wobei der einstellbare Schwellwertschalter über einen regelbaren Widerstand verbunden ist.

Hierdurch wird erreicht, daß der Schwellwertschalter sowohl zu einer Grobeinstellung als auch zu einer Feinregelung des Lichtbogens zwischen der Aktiv-Elektrode und dem Gewebe verwendet werden kann. Die Grobeinstellung, die bevorzugt auf einfache Weise über einen regelbaren Widerstand erfolgt, wird in Abhängigkeit von dem gewünschten Einsatz des elektrochirurgischen HF-Gerätes vorgenommen. Als Einstellkriterien dienen dabei z.B. der gewünschte Verscharfungsgrad beim Schneiden, der Einsatz für eine Kontakt-Koagulation, für Fulguration, usw.. Ausgehend von dem durch die Grobeinstellung ausgewählten mittleren Leistungspegel erfolgt dann eine Feinregelung des HF-Generators in Abhängigkeit von der am Ausgang der proportionalen Entladeerkennungsschaltung vorliegenden Spannung.

Bei einer bevorzugten Weiterbildung der Erfindung ist vorgesehen, daß die Vergleichsschaltung einen Mikrorechner aufweist, dem ein Sollwert für das Ausgangssignal der Entladeerkennungsschaltung eingebbar ist. Durch die erfindungsgemäße Verwendung eines Mikrorechners als Vergleichsschaltung wird erreicht, daß verschiedene Regelvarianten für den HF-Generator ausgeführt werden können, ohne daß wesentliche Eingriffe in den Aufbau des erfindungsgemäßen HF-Gerätes erforderlich wären.

Bei einer weiteren Ausgestaltung der Erfindung ist vorgesehen, daß dem Mikrorechner ein Reduzierungswert für den Sollwert eingebbar ist und daß dem Mikrorechner ein das Einschwingen des Regelkreises anzeigendes Signal zugeführt ist, um eine Umschaltung des Sollwerts für das Ausgangssignal der Entladeerkennungsschaltung vom eingegebenen Sollwert auf einen reduzierten Sollwert zu bewirken, sobald der Regelkreis das erste Mal eingeschwungen hat.

Durch die erfindungsgemäße Überwachung des Einschwingens des Regelkreises wird erreicht, daß zunächst während des Einschwingens die Regelung des HF-Generators in Abhängigkeit vom eingegebenen Sollwert erfolgt, um anschließend in Abhängigkeit vom reduzierten Sollwert vorgenommen zu werden. Dadurch wird neben einer optimalen Glimmentladung zwischen der Aktiv-Elektrode und dem Gewebe in besonders vorteilhafter Weise ein optimales Anschneideverhalten sichergestellt.

Um die Sicherheit des erfindungsgemäßen HF-Gerätes zu verbessern, ist bei einer bevorzugten Weiterbildung der Erfindung vorgesehen, daß zwischen der Entladeerkennungsschaltung und der Vergleichsschaltung eine Trennschaltung zur galvanischen Trennung vorgesehen ist.

Dabei ist bei einer praktischen Ausgestaltung der Erfindung vorgesehen, daß die Trennschaltung eine spannungsmäßige galvanische Trennung von mindestens 4 kV bewirkt, wobei die Trennschaltung einen Optokoppler aufweist, dem ein Verstärker nachgeschaltet ist.

Eine weitere Verbesserung der Erfindung ergibt sich, wenn in der Zuleitung zur Neutral-Elektrode ein zweiter Antifaradisationskondensator vorgesehen ist. Hierdurch wird zunächst erreicht, daß die Gleichstromanteile im Hochfrequenzkreis von der Entladeerkennungsschaltung noch genauer erfaßt werden können, so daß sich eine äußerst präzise Regelung des HF-Generators bzw. des Lichtbogens ergibt. Gleichzeitig bewirkt der zweite Antifaradisationskondensator, daß selbst bei einem Isolationsdefekt im HF-Generator der Patient, der unter Verwendung des erfindungsgemäßen HF-Geräts behandelt wird, nicht direkt mit der Betriebsspannung des HF-Geräts, die am HF-Generator anliegt, in Verbindung kommen kann.

Eine weitere Ausgestaltung der Erfindung zeichnet sich dadurch aus, daß zwischen den Antifaradisationskondensatoren und der Aktiv- und Neutral-Elektrode eine HF-Ableitstrom-Begrenzungsschaltung in den Zuleitungen vorgesehen ist, wobei die HF-Ableitstrom-Begrenzungsschaltung eine Drossel mit großer Koppelkapazität aufweist.

Hierdurch lassen sich insbesondere negative Auswirkungen einer Veränderung des kapazitiven Verhaltens vermeiden, da durch die erfindungsgemäß vorgesehene große Koppelkapazität der HF-Ableitstrom-Begrenzungsschaltung Kapazitätsänderungen, die durch Leitungskapazitäten der anzuschließenden Elektroden hervorgerufen werden, praktisch nicht ins Gewicht fallen.

Ferner wirkt die erfindungsgemäß vorgesehene HF-Ableitstrom-Begrenzungsschaltung als Filter für Schwingungen, die höhere Frequenzen aufweisen als die Arbeitsfreguenz des Generators.

Besonders vorteilhaft ist es, wenn die Koppelkapazität der Drossel 200 pF bis 400 pF, vorzugsweise 250 pF-350 pF, insbesondere 300 pF beträgt.

Die Erfindung wird im folgenden beispielsweise anhand der Zeichnung näher erläutert; in dieser zeigt:
- Fig. 1: ein schematisches Blockschaltbild eines elektrochirurgischen HF-Gerätes und
- Fig. 2: ein schematisches Blockschaltbild eines weiteren elektrochirurgischen HF-Gerätes.

In den Figuren der Zeichnung sind einander entsprechende Schaltungsbauteile mit gleichen Bezugszeichen bezeichnet.

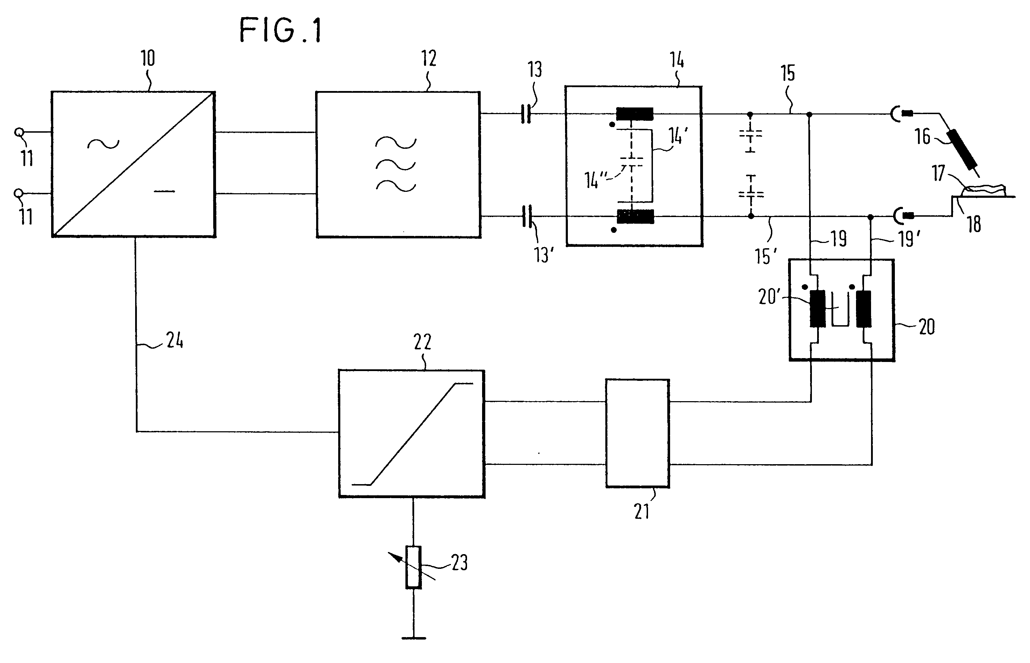

Nach Fig. 1 weist das elektrochirurgische HF-Gerät ein Schaltnetzteil 10 mit einer nicht näher dargestellten Regelschaltung auf, dessen Wechselspannungsseite über Anschlußklemmen 11 mit einem elektrischen Netz verbindbar ist und dessen Gleichspannungsseite mit einem HF-(Hochfreguenz-)Generator 12 verbunden ist. Der HF-Generator 12 ist über einen ersten und einen zweiten Antifaradistionskondensator 13, 13′ mit einer HF-Ableitstrom-Begrenzungsschaltung 14 verbunden, an die über eine erste Leitung 15 eine Aktiv-Elektrode 16 zum Schneiden oder Koagulieren von Gewebe 17 anschließbar ist. Über eine zweite Leitung 15′ ist eine inaktive Neutral-Elektrode 18 an die HF-Ableitstrom-Begrenzungsschaltung 14 anschließbar, die mit dem Gewebe 17 in großflächigen Kontakt bringbar ist.

An die Leitungen 15, 15′ ist über Anschlußleitungen 19 bzw. 19′ eine proportionale Entladeerkennungsschaltung 20 angeschlossen, die eine als Gleichspannungsquelle wirkende stromkompensierte Drossel 20′ umfaßt. Die Ausgänge der Entladeerkennungsschaltung sind mit einer Trennschaltung 21 verbunden, die beispielsweise einen Optokoppler mit anschließendem Verstärker umfaßt, um eine galvanische Trennung, die spannungsmäßig mindestens 4 kV beträgt, zwischen dem Netz und einem Patienten zu bewirken. Die Ausgangsseite der Trennschaltung 21 ist mit einem Schwellwertschalter 22 verbunden, der über einen an Masse liegenden regelbaren Widerstand 23 einstellbar ist. Das Ausgangssignal des regelbaren Schwellwertschalters 22 ist über eine Steuerleitung 24 an das Schaltnetzteil 10 angelegt, um die Regeleinrichtung des Schaltnetzteils zu steuern.

Die HF-Ableitstrom-Begrenzungsschaltung 14 umfaßt eine Drossel 14′ mit einer großen Koppelkapazität, die in der Größenordnung von 300 pF liegt und die in der Zeichnung durch den gestrichelt dargestellten Kondensator 14˝ angedeutet ist.

Im folgenden wird die Funktion des beschriebenen elektrochirurgischen HF-Gerätes erläutert.

Zum Schneiden von Gewebe 17, das mit der Neutral-Elektrode 18 in Kontakt ist, mit der Aktiv-Elektrode 16 liefert der HF-Generator 12, der eine hochfrequente Wechselspannung von etwa 500 kHz erzeugt, die zur Bildung eines Lichtbogens zwischen der Aktiv-Elektrode 16 und dem Gewebe 17 erforderliche HF-Leistung über die Antifaradisationskondensatoren 13, 13′, die HF-Ableitstrom-Begrenzungsschaltung und die Leitungen 15, 15′ zu den Elektroden 16, 18.

Der Antifaradisationskondensator 13 in der Zuleitung zur Aktiv-Elektrode 16 besitzt eine maximale Kapazität von 5 nF und ist zur Vermeidung faradischer Effekte unerläßlich. Um die Sicherheit des elektrochirurgischen HF-Gerätes weiter zu erhöhen und um gleichzeitig die Unterdrückung faradischer Effekte zu verbessern, ist der zweite Antifaradisationskondensator 13′ in der Zuleitung zur Neutral-Elektrode vorgesehen, der eine maximale Kapazität von 22 nF annehmen kann. Durch die Verwendung der beiden Antifaradisationskondensatoren wird sichergestellt, daß selbst bei einem Isolationsdefekt im HF-Generator 12 der Patient mit der Betriebsspannung des Schaltnetzteils 10 nicht in Berührung kommen kann.

Die Drossel 14′ der HF-Ableitstrom-Begrenzungsschaltung 14 verhindert infolge ihrer großen Koppelkapazität 14˝, daß sich das kapazitive Verhalten des HF-Gerätes durch den Anschluß von Leitungen bzw. der Elektroden 16, 18 wesentlich ändert, da die Koppelkapazität 14˝ der Drossel 14 groß gegenüber der Leitungskapazität ist. Hierdurch werden negative Auswirkungen durch eine Veränderung des kapazitiven Verhaltens bei der Anwendung in der Hochfrequenz-Chirurgie vermieden.

Zusätzlich wirkt die Drossel 14′ der HF-Ableitstrom-Begrenzungsschaltung 14 als Filter für Schwingungen, deren Frequenz höher liegt als die Arbeitsfreguenz des HF-Generators 12.

Bei der Bildung eines Lichtbogens, der eine unerläßliche Voraussetzung zum Schneiden von Gewebe 17 darstellt, an der Aktiv-Elektrode entsteht ein unsymmetrischer Entladevorgang für die positive und negative Halbwelle der vom HF-Generator 12 gelieferten hochfrequenten Wechselspannung, da die Austrittsarbeit der Elektronen aus dem Gewebe 17 eine andere ist, als die Austrittsarbeit der Elektronen aus der Aktiv-Elektrode 16. Hierdurch ergeben sich im Hochfrequenzkreis Gleichstromanteile, die als faradische Effekte bekannt sind. Dabei sind die Gleichstromanteile ein Maß für die Größe des Lichtbogens zwischen der Aktiv-Elektrode 16 und dem Gewebe 17.

Die stromkompensierte Drossel 20′ der Entladeerkennungsschaltung 20 wirkt infolge ihrer bei unsymmetrischen Schwingungen, die durch den unsymmetrischen Entladevorgang hervorgerufen werden, voll wirksamen Impedanz als hervorragende Sperre für HF-Schwingungen. Gleichzeitig stellt sie eine Gleichspannungsquelle dar, deren Gleichspannung durch die Unsymmetrie des Entladevorgangs zwischen der Aktiv-Elektrode 16 und dem mit der Neutral-Elektrode 18 in Kontakt stehendem Gewebe 17 bestimmt ist. Diese Gleichspannung kann auf der Sekundärseite der stromkompensierten Drossel 20′, also auf der Ausgangsseite der Entladeerkennungsschaltung 20 gemessen werden und als Regelgröße für die Leistungsregelung des HF-Generators 12 verwendet werden.

Dazu wird die Gleichspannung am Ausgang der Entladeerkennungsschaltung 20 über die Trennschaltung 21 an den regelbaren Schwellwertschalter 22 angelegt, der je nach Einstellung der Spannungsschwelle mittels des regelbaren Widerstandes 23 die Leistungsregelung des HF-Generators 12 über die Regelschaltung des Schaltnetzteils 10 bewirkt und je nach Lichtbogengröße, also je nach Größe der am Ausgang der Entladeerkennungsschaltung 20 vorliegenden Spannung die Ausgangsleistung des HF-Generators 12 aufwärts oder abwärts regelt.

Hierbei dient der Schwellwertschalter 22 sowohl der Grobals auch der Feineinstellung.

Die Grobeinstellung erfolgt dabei durch die Einstellung der Spannungsschwelle, wodurch ein mittlerer Ausgangsleistungspegel des HF-Generators 12 gegeben ist. Durch die von der Entladeerkennungsschaltung 20 erfaßte Spannung, die durch die Trennschaltung 21 an den Schwellwertschalter 22 übertragen wird, wird dann innerhalb eines bestimmten Bereichs die Regelschaltung des Schaltnetzteils 10 beeinflußt, um so die Lichtbogengröße zu regeln.

Das beschriebene elektrochirurgische HF-Gerät läßt sich auch in vorteilhafter Weise für eine Kontakt-Koagulation einsetzen, bei der zwischen der Aktiv-Elektrode 16 und dem Gewebe 17 ein direkter ohmscher Kontakt besteht. Bei der Koagulation sollen bekanntlich keine Funken entstehen. Derartige Funken bewirken ebenso wie der Lichtbogen beim Schneiden einen unsymmetrischen Entladevorgang, so daß eine am Ausgang der Entladeerkennungsschaltung 20 erfaßte Gleichspannung während der Kontakt-Koagulation anzeigt, daß Funken beim Koagulieren entstanden sind. Das Auftreten einer Gleichspannung am Ausgang der Entladeerkennungsschaltung 20 kann somit zum Abschalten des HF-Generators benutzt werden. Hierdurch läßt es sich auf einfache und zuverlässige Weise erreichen, daß bereits bei der Bildung erster Funken der HF-Generator 12 abgeschaltet wird. Wird nun eine entsprechende Leistungscharakteristik des HF-Generators 12 gewählt, kann auf diese Weise eine vollautomatisch geregelte Koagulation realisiert werden.

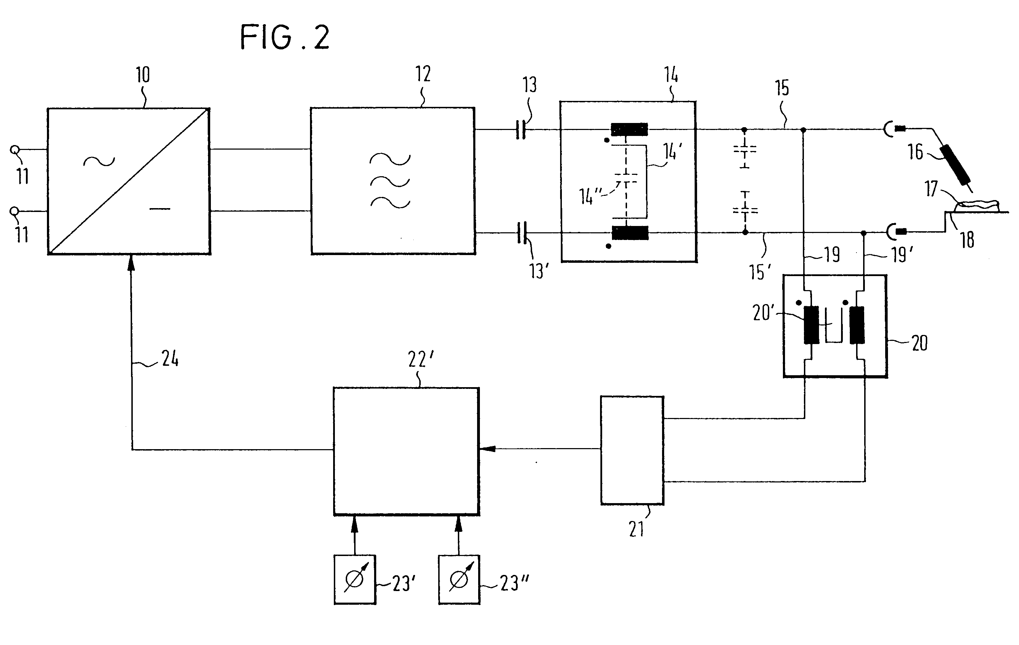

Das in Fig. 2 dargestellte elektrochirurgische HF-Gerät unterscheidet sich von dem anhand von Fig. 1 beschriebenen HF-Gerät im wesentlichen nur dadurch, daß anstelle eines Schwellwertschalters ein Mikrorechner 22′ vorgesehen ist, dem das Ausgangssignal der Trennschaltung 21 zugeführt ist. Der Mikrorechner 22′ ist mit einer ersten Einstellvorrichtung 23′, mit der ein Sollwert für das Ausgangssignal der Trennschaltung 21 eingebbar ist, und mit einer zweiten Einstellvorrichtung 23˝ verbunden, mit der ein Reduzierungswert für den Sollwert als Prozentzahl in den Mikrorechner 22′ eingegeben werden kann.

Im folgenden wird die Funktionsweise des in Fig. 2 dargestellten elektrochirurgischen HF-Gerätes beschrieben, soweit sie sich von der des anhand von Fig. 1 beschriebenen HF-Gerätes unterscheidet:

Vor der Inbetriebnahme des HF-Gerätes zum Schneiden wird zunächst mittels der ersten Einstellvorrichtung 23′ ein Sollwert für die von der Entladeerkennungsschaltung 20 erfaßte Gleichspannung zwischen den Elektroden 16, 18 eingestellt. Zusätzlich wird für den Reduzierungswert eine Prozentzahl mittels der zweiten Einstellvorrichtung 23˝ in den Mikrorechner 22′ eingegeben.

Zu Beginn des Schneidens von Gewebe 17 mittels der Aktiv-Elektrode 16 wird zunächst der Schneidvorgang entsprechend dem durch die erste Einstellvorrichtung 23′ eingegebenen Sollwert überwacht und geregelt. Gleichzeitig erfaßt der Mikrorechner 22′ dabei wie der beschriebene Regelkreis einschwingt. Sobald dabei festgestellt wird, daß der Regelkreis zum ersten Mal eingeschwungen ist, reduziert der Mikrorechner 22 den eingegebenen Sollwert entsprechend dem Reduzierungswert und führt die weitere Regelung des Schneidvorgangs in Abhängigkeit vom reduzierten Sollwert durch. Auf diese Weise läßt sich neben dem optimalen Regeln des Schneidvorgangs auch die Regelung des HF-Gerätes beim Anschneiden wesentlich verbessern.

## Patentansprüche

1. Elektrochirurgisches Hochfrequenzgerät mit einem HF-(Hochfrequenz-)Generator (12), an den eine mit einem Gewebe (17) in Kontakt bringbare Neutral-Elektrode (18) und eine Aktiv-Elektrode (16) für chirurgische Eingriffe über Zuleitungen (15, 15') anschließbar ist und mit einer Regelschaltung, die zur Erfassung eines Gleichspannungsanteils im HF-(Hochfrequenz-)Kreis mit zumindest einer der Elektroden verbunden ist und die ein Ausgangssignal (24) zum Steuern des HF-Generators liefert,
dadurch **gekennzeichnet,** daß
die Regelschaltung eine auf unsymmetrische Entladungsvorgänge an der Aktivelektrode ansprechende und ein der Unsymmetrie zwischen dem bei der positiven und negativen Halbwelle des HF-Generators auftretenden Entladungsvorgang entsprechendes Signal abgebende proportionale Entladungserkennungsschaltung (20) umfaßt, deren Eingangsseite mit den Elektroden verbunden ist und deren Ausgang einen Eingang einer den HF-Generator (12) über ein regelbares Schaltnetzteil (10) in der Leistung steuernden Vergleichsschaltung (22, 22') beaufschlagt, wobei zumindest ein Eingang der Entladeerkennungsschaltung (20) durch einen in der Zuleitung zur Aktivelektrode vorgesehenen Antifaradisationskondensator (13) galvanisch von den den Patienten mit Spannung beaufschlagenden Ausgängen des HF-Generators (12) getrennt und ein zweiter Eingang entweder geerdet und/oder durch einen in der Zuleitung zur Elektrode vorgesehenen Antifaradisationskondensator (13') galvanisch von den den Patienten mit Spannung beaufschlagenden Ausgängen des HF-Generators (12) getrennt ist.

2. HF-Gerät nach Anspruch 1,
dadurch **gekennzeichnet,**
daß die Entladeerkennungsschaltung (20) eine stromkompensierte Drossel (20') umfaßt, die als Gleichspannungsquelle wirkt.

3. HF-Gerät nach Anspruch 1 oder 2,
dadurch **gekennzeichnet,**
daß die Vergleichsschaltung ein einstellbarer Schwellwertschalter (22) ist, wobei insbesondere der einstellbare Schwellwertschalter (22) über einen regelbaren Widerstand (23) mit Masse verbunden ist.

4. HF-Gerät nach Anspruch 1 oder 2,
dadurch **gekennzeichnet,** daß die Vergleichsschaltung einen Mikrorechner (22') aufweist, dem ein Sollwert für das Ausgangssignal der Entladeerkennungsschaltung (20) eingebbar ist, wobei insbesondere dem Mikrorechner (22') ein Reduzierungswert für den Sollwert eingebbar ist und daß dem Mikrorechner (22') ein das Einschwingen des Regelkreises anzeigendes Signal zugeführt ist, um eine Umschaltung des Sollwerts für das Ausgangssignal der Entladeerkennungsschaltung vom eingegebenen Sollwert auf einen reduzierten Sollwert zu bewirken, sobald der Regelkreis das erste Mal eingeschwungen hat.

5. HF-Gerät nach einem der vorhergehenden Ansprüche,
dadurch **gekennzeichnet,**
daß zwischen der Entladeerkennungsschaltung (20) und der Vergleichsschaltung (22) eine Trennschaltung (21) zur galvanischen Trennung vorgesehen ist, wobei insbesondere die Trennschaltung (21) eine spannungsmäßige galvanische Trennung von mindestens 4 kV bewirkt.

6. HF-Gerät nach Anspruch 5,
dadurch **gekennzeichnet,**
daß die Trennschaltung (21) einen Optokoppler aufweist, dem ein Verstärker nachgeschaltet ist.

7. HF-Gerät nach einem der vorhergehenden Ansprüche,
dadurch **gekennzeichnet,**
daß in der Zuleitung (15') zur Neutral-Elektrode (18) ein zweiter Antifaradisationskondensator (13') vorgesehen ist.

8. HF-Gerät nach einem der vorhergehenden Ansprüche,
dadurch **gekennzeichnet,**
daß zwischen den Antifaradisationskondensatoren (13, 13') und der Aktiv- und Neutral-Elektrode (16 bzw. 18) eine HF-Ableitstrom-Begrenzungsschaltung (14) in den Zuleitungen (15, 15') vorgesehen ist.

9. HF-Gerät nach Anspruch 8,
dadurch **gekennzeichnet,**
daß die HF-Ableitstrom-Begrenzungsschaltung (14) eine Drossel (14') mit großer Koppelkapazität aufweist.

10. HF-Gerät nach Anspruch 9,
dadurch **gekennzeichnet,**
daß die Koppelkapazität der Drossel (14') 200 pF bis 400 pF, vorzugsweise 250 pF bis 350 pF, insbesondere 300 pF beträgt.

## Claims

1. An electrosurgical high-frequency instrument having an RF (high frequency) generator (12) to which can be connected, via electrical feedlines (15, 15'), a neutral electrode (18) which can be brought into contact with a tissue (17) and an active electrode (16) for surgical treatment, and having a control circuit which is connected to at least one of the electrodes for sensing a direct current component in the RF (high frequency) circuit and which furnishes an output signal (24) for controlling the RF generator, characterised in that the control circuit includes a proportional discharge recognition circuit (20) which responds to an asymmetrical discharge process at the active electrode and transmits a signal corresponding to the asymmetry between the positive and negative half wave of the discharge process arising at the RF generator, with the input end of the recognition circuit being connected to the electrodes and with the output end of the recognition circuit being connected to a comparator circuit (22, 22') controlling the power of the RF generator (12) via a regulatable switching power supply (10), with at least one input of the discharge recognition circuit (20) being galvanically separated from the outputs of the RF generator (12) which subject the patient to voltage by an anti-faradisation capacitor (13) provided in the feedline to the active electrode and with a second input being either earthed and/or galvanically separated from the outputs of the RF generator (12) which subject the patient to voltage by an anti-faradisation capacitor (13') provided in the feedline to the electrode.

2. RF instrument according to claim 1, characterised in that said discharge recognition circuit (20) includes a current compensated choke (20') acting as a source of DC voltage.

3. RF instrument according to claim 1 or claim 2, characterised in that the comparator circuit is an adjustable threshold switch (22) with the adjustable threshold switch (22) in particular being connected to earth via a variable resistor (23).

4. RF instrument according to claim 1 or claim 2, characterised in that the comparator circuit has a microcomputer (22') into which a desired value for the output signal of the discharge recognition circuit (20) can be fed, with it in particular being possible to feed a reduction value for the desired value into the microcomputer (22'); and in that a signal indicating tuning of the control circuit is supplied to the microcomputer (22') to cause a switch over of the desired value for the output signal of said discharge recognition circuit from the input desired value to a reduced desired value as soon as the control circuit has been tuned for the first time.

5. RF instrument according to one of the previous claim 3, characterised in that a decoupling circuit (21) for galvanic separation is provided between the discharge recognition circuit (20) and the comparator circuit (22), with the decoupling circuit (21) in particular bringing about a voltage-wise galvanic separation of at least 4 kV.

6. RF instrument according to claim 5, characterised in that said decoupling circuit (21) has a optocoupler followed by an amplifier.

7. RF instrument according to one of the previous claims, characterised in that a second anti-faradisation capacitor (13') is provided in the feedline (15') to said neutral electrode (18).

8. RF instrument according to one of the previous claims, characterised in that an RF drain current limiter circuit (14) is incorporated in said feedlines (15, 15') between the anti-faradization capacitors (13, 13') and the active and neutral electrodes (16 and 18 resp.).

9. RF instrument according to claim 8, wherein the RF drain current limiter circuit (14) features a choke (14') of high coupling capacity.

10. RF instrument according to claim 9, wherein the coupling capacity of said choke (14') amounts to 200 pF to 400 pF, is preferably 250 pF - 350 pF, and in particular amounts to 300 pF.j

## Revendications

1. Appareil pour l'électro-chirurgie à haute fréquence, comprenant un générateur haute fréquence (12), auquel peuvent se raccorder, par l'intermédiaire de conduites (15, 15'), une électrode neutre (18) pouvant être mise en contact avec un tissu (18) et une électrode active (16) pour interventions chirurgicales, et comprenant un circuit de régulation qui, pour détecter une part de tension continue dans le circuit à haute fréquence, est relié à au moins l'une des électrodes et délivre un signal de sortie (24) destiné à piloter le générateur haute fréquence, caractérisé en ce que le circuit de régulation comprend un circuit proportionnel de détection de décharge (20) réagissant à des processus de décharges asymétriques au niveau de l'électrode active et délivrant un signal correspondant à l'asymétrie entre les processus de décharge apparaissant au niveau des demi-ondes positive et négative du générateur haute fréquence, circuit dont le côté d'entrée est relié aux électrodes et dont la sortie alimente une entrée d'un circuit comparateur (22, 22') pilotant la puissance du générateur haute fréquence (12) par l'intermédiaire d'un élément de réseau combinatoire (10) réglable, au moins une entrée du circuit de détection de décharge (20) étant, par un condensateur d'antifaradisation (13) prévu sur la conduite menant à l'électrode active, séparée de façon galvanique des sorties du générateur haute fréquence (12) appliquant une tension au patient, et une deuxième entrée étant mise à la terre et/ou séparée de façon galvanique des sorties du générateur haute fréquence (12) appliquant une tension au patient, par un condensateur d'antifaradisation (13') prévu sur la conduite menant à l'électrode.

2. Appareil haute fréquence selon la revendication 1, caractérisé en ce que le circuit de détection de décharge (20) comprend une bobine réactance (20') de compensation de courant, qui agit en tant que source de tension continue.

3. Appareil haute fréquence selon la revendication 1 ou 2, caractérisé en ce que le circuit comparateur est un commutateur à valeur de seuil (22) réglable, en particulier le commutateur à valeur de seuil (22) réglable étant relié à la masse par l'intermédiaire d'une résistance réglable (22).

4. Appareil haute fréquence selon la revendication 1 ou 2, caractérisé en ce que le circuit comparateur présente un microcalculateur (22'), dans lequel peut être introduit une valeur de consigne pour le signal de sortie du circuit de détection de décharge (20), en particulier une valeur de réduction pour la valeur de consigne pouvant être introduite dans le microcalculateur (22'), et en ce qu'un signal indiquant la stabilisation du circuit de régulation est introduit dans le microcalculateur pour provoquer une commutation de la valeur de consigne pour le signal de sortie du circuit de détection de décharge, de la valeur de consigne introduite à une valeur de consigne réduite, dès que le circuit de régulation s'est stabilisé la première fois.

5. Appareil haute fréquence selon l'une des revendications précédentes, caractérisé en ce qu'entre le circuit de détection de décharge (20) et le circuit comparateur (22), il est prévu un circuit séparateur (21) pour la séparation galvanique, en particulier le circuit séparateur (21) provoquant une séparation galvanique en tension d'au moins 4 kV.

6. Appareil haute fréquence selon la revendication 5, caractérisé en ce que le circuit séparateur (21) présente un coupleur optoélectronique qui est suivi d'un amplificateur.

7. Appareil haute fréquence selon l'une des revendications précédentes, caractérisé en ce que, sur la conduite (15') menant à l'électrode neutre (18), il est prévu un deuxième condensateur d'antifaradisation (13').

8. Appareil haute fréquence selon l'une des revendications précédentes, caractérisé en ce qu'entre les condensateurs d'antifaradisation (13, 13') et les électrodes active et neutre (16, resp. 18), il est prévu sur les conduites (15, 15') un circuit de limitation de courant de fuite haute fréquence (14).

9. Appareil haute fréquence selon la revendication 8, caractérisé en ce que le circuit de limitation de courant de fuite haute fréquence (14) présente une bobine réactance (14') à forte capacité de couplage.

10. Appareil haute fréquence selon la revendication 9, caractérisé en ce que la capacité de couplage de la bobine réactance (14') vaut 200 pF à 400 pF, de préférence 250 pF à 350 pF, en particulier 300 pF.
